(19) Europäisches Patentamt
European Patent Office
Office européen des brevets

(11) Numéro de publication: **0 069 623**
**B1**

# (12) FASCICULE DE BREVET EUROPEEN

(45) Date de publication du fascicule du brevet: **10.04.85**

(21) Numéro de dépôt: **82401126.6**

(22) Date de dépôt: **21.06.82**

(51) Int. Cl.⁴: **C 07 D 513/04,** A 61 K 31/415, A 61 K 31/505 // (C07D513/04, 277:00, 235:00),(C07D513/04, 277:00, 239:00)

(54) **Dérivés phénéthyles de thiazole, leur préparation et leur application en thérapeutique.**

(30) Priorité: **03.07.81 FR 8113078**

(43) Date de publication de la demande: **12.01.83 Bulletin 83/2**

(45) Mention de la délivrance du brevet: **10.04.85 Bulletin 85/15**

(84) Etats contractants désignés: **AT BE CH DE FR GB IT LI LU NL SE**

(56) Documents cités:
**EP - A - 0 038 731**
**FR - A - 2 014 048**

**Le dossier contient des informations techniques présentées postérieurement au dépôt de la demande et ne figurant pas dans le présent fascicule.**

(73) Titulaire: **SYNTHELABO, 58, rue de la Glacière, F-75621 Paris Cedex 13 (FR)**

(72) Inventeur: **Bigg, Dennis, 5, Parc de Diane, F-78350 Jouy en Josas (FR)**

(74) Mandataire: **Thouret-Lemaitre, Elisabeth et al, Service Brevets - SYNTHELABO 58, rue de la Glacière, F-75621 Paris Cedex 13 (FR)**

**EP 0 069 623 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne des dérivés phénéthylés de thiazole, leur préparation et leur application en thérapeutique.

Les composés de l'invention répondent à la formule (I)

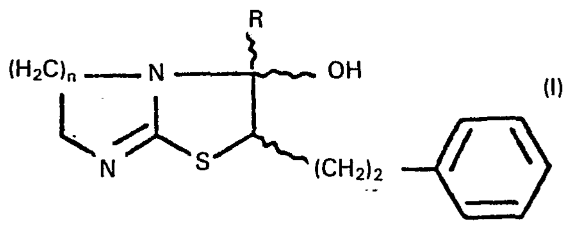

dans laquelle
n est 1 ou 2,
R est un radical naphthyle, phényle ou phényle portant un ou plusieurs substituants choisis dans le groupe constitué par les radicaux $C_{1-4}$alkyle, $C_{1-4}$alcoxy méthylènedioxy, $CF_3$ et les atomes d'halogène.

Des composés de structure analogue, essentiellement anorexigènes et stimulants, sont déjà connus par la demande de brevet français n° 2 014 048.

Les composés (I) comportent des centres d'asymétrie et peuvent exister sous la forme d'isomères optiques.

Les sels que forment les composés (I) avec les acides pharmaceutiquement acceptables font partie de l'invention.

Les significations de R sont plus particulièrement les radicaux naphtyle, phényle ou phényle portant un ou deux substituants choisis parmi les radicaux méthyle, méthoxy, méthylènedioxy, $CF_3$ et les atomes de chlore, de brome et de fluor.

Parmi les composés (I) sont préférés ceux pour lesquels R est le radical phényle portant en position 3 ou en positions 3 et 4 un ou deux atomes de chlore ou groupes méthoxy.

Selon l'invention on peut préparer les composés de l'invention selon le schéma réactionnel suivant:

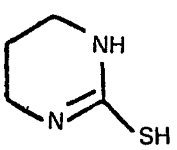

ou

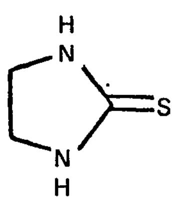

(IIbis) (II)

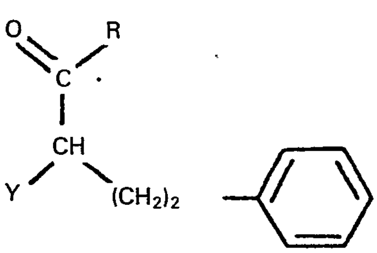

(III)

Dans les composés de départ (III) Y est un atome de brome ou de chlore ou tout groupe labile.

La réaction entre les composés (II) ou (IIbis) et (III) est effectuée dans un solvant approprié, tel que l'acétone ou un alcool, par exemple l'isopropanol.

Lés cétones qui mènent aux composés de départ III sont obtenues soit par la réaction classique du nitrile avec le réactif de Grignard et ensuite l'hydrolyse, par exemple selon la méthode décrite par W.J. Humphlett, M.J. Weiss et C.R. Hauser, J. Am. Chem. Soc. 70, 4020, (1948), soit par réaction du chlorure d'acide avec le réactif de Grignard dans du tétrahydrofuranne à —78°C, par exemple selon la méthode décrite par F. Sato, M. Inoue, K. Oguro et M. Sato, Tetrahedron Letters n° 44, pp. 4303-4306, (1979), soit par oxydation de l'alcool correspondant.

Les exemples suivants illustrent l'invention.

Les analyses et les spectres IR et RMN confirment la structure des composés.

*Exemple 1*

*(Phénéthyl)-2 phényl-3 tétrahydro-2,3,5,6 imidazo[2,1-b] thiazolol-3 et son chlorhydrate*

[n = 1, R = 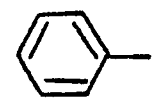 ]

On ajoute, en une seule fois, 25 g (0,082 mole) d'$\alpha$-bromophényl-phénylpropylcétone en solution dans 200 ml d'acétone à 6,3 g (0,0618 mole) d'imidazolidinethione-2 en solution dans 1 l d'acétone.

On agite pendant 20 heures. On filtre et rince plusieurs fois à l'acétone puis une fois à l'éther. On sèche sous vide. On obtient le bromhydrate du composé qui fond à 140-141°C.

On le reprend par de l'eau et $CH_2Cl_2$. On agite et alcalinise avec $Na_2CO_3$. On extrait avec $CH_2Cl_2$ puis lave à l'eau, sèche sur $MgSO_4$, filtre et concentre. On reprend le composé dans de l'éther diisopropylique, filtre et rince avec ce solvant.

La base obtenue fond à 148-150°C.

On peut préparer le chlorhydrate en faisant passer un courant de HCl gazeux dans 5,6 g (0,017 mole) de base en solution dans un mélange $CH_2Cl_2$/MeOH.

F = 138-139°C.

*Exemple 2*

*Phénéthyl-2 (chloro-3 phényl)-3 tétrahydro-2,3,5,6 imidazo[2,1-b] thiazolol-3 et son chlorhydrate*

[n = 1, R = 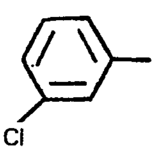 ]

On ajoute 28 g (0,083 mole) d'$\alpha$-bromo-(chloro-3 phényl)-phénylpropylcétone en solution dans 200 ml d'acétone à 4,3 g (0,042 mole) d'imidazolidinethione-2 en solution dans 570 ml d'acétone. On filtre le produit précipité et le rince à l'acétone puis à l'éther.

On libère la base en reprenant le bromhydrate dans un mélange eau/chloroforme et en ajoutant du carbonate de sodium jusqu'à pH 9.

Après extraction avec du chloroforme et lavage à l'eau, on sèche le produit sur $MgSO_4$, filtre et concentre. On reprend le produit cristallisé dans de l'éther, filtre et rince avec de l'éther. On fait passer un courant de gaz chlorhydrique dans la solution de 9,1 g de poudre dans un mélange 1/1 de $MeOH/CH_2Cl_2$. On filtre le chlorhydrate formé, le rince à l'éther et le sèche sous vide.

F = 134-135°C.

*Exemple 3*

*Phénéthyl-2 (chloro-3 phényl)-3 tétrahydro-2,3,6,7 5H-thiazolo[3,2-a]pyrimidinol-3 et son chlorhydrate*

[n = 2, R = 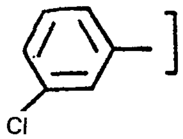 ]

On ajoute 28 g (0,083 mole) d'α-bromo-(chloro-3 phényl)-phénylpropylcétone en solution dans 200 ml d'acétone à 4,9 g (0,042 mole) de tétrahydropyrimidinethiol-2 en solution dans 800 ml d'acétone. On agite pendant une journée, filtre et rince à l'acétone puis à l'éther.

On reprend le bromhydrate formé dans un mélange eau/$CHCl_3$ et libère la base par addition de $Na_2CO_3$. On extrait avec du chloroforme la base et la lave à l'eau, sèche, filtre et concentre. On obtient une huile que l'on reprend dans de l'éther.

Le produit cristallise. On fait passer un courant de HCl gazeux dans une solution de la base dans un mélange $MeOH/CH_2Cl_2$; dans un bain d'eau glacée. Le chlorhydrate précipite par addition d'éther. On filtre, rince à l'éther et sèche sous vide.

F = 177-178°C.

Dans le tableau suivant sont représentés les composés de l'invention préparés à titre d'exemples.

TABLEAU

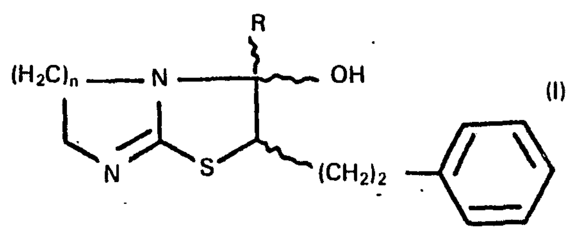

(I)

| Composé | n | R | Forme | F(°C) |
|---|---|---|---|---|
| 1 | 1 | $C_6H_5$ | base libre | 148-150 |
| 2 | 1 | $C_6H_5$ | HCl | 138-9 |
| 3 | 1 | 4-Cl-$C_6H_4$ | base libre | 161-2 |
| 4 | 1 | 3-Cl-$C_6H_4$ | HCl | 134-5 |
| 5 | 1 | 3,4-$Cl_2$-$C_6H_3$ | HBr | 159-160 |
| 6 | 1 | 2-Me-$C_6H_4$ | HCl | 139-140 |
| 7 | 1 | 2-Cl-$C_6H_4$ | HCl | 155-6 |
| 8 | 1 | 2,3-$Me_2$-$C_6H_3$ | HBr | 142-3 |
| 9 | 1 | 2-F-$C_6H_4$ | HCl | 131-2 |
| 10 | 1 | 3-$CF_3$-$C_6H_4$ | HBr | 144-5 |
| 11 | 1 | α-naphtyle | HBr | 148-148,5 |

TABLEAU (suite)

| Composé | n | R | Forme | F(°C) |
|---|---|---|---|---|
| 12 | 1 | 2,4-$Cl_2$-$C_6H_3$ | HBr | 138-140 |
| 13 | 1 | 2-MeO-$C_6H_4$ | HCl | 153-153,5 |
| 14 | 1 | 4-Br-$C_6H_4$ | HCl | 145-6 |
| 15 | 1 | 3,4-$(MeO)_2$-$C_6H_3$ | HCl | 132-3 |
| 16 | 1 | 3,4-$(OCH_2O)$-$C_6H_3$ | HCl | 139-140 |
| 17 | 2 | $C_6H_5$ | base libre | 154-5 |
| 18 | 2 | 4-Cl-$C_6H_4$ | base libre | 105-7 |
| 19 | 2 | 3-MeO-$C_6H_4$ | HBr | 185-6 |
| 20 | 2 | 2-Me-$C_6H_4$ | HBr | 146-7 |
| 21 | 2 | 2-Cl-$C_6H_4$ | HBr | 199-200 |
| 22 | 2 | 3-Cl-$C_6H_4$ | HCl | 177-8 |
| 23 | 2 | 3,4-$Cl_2$-$C_6H_3$ | HCl | 198-9 |
| 24 | 2 | 3,4-$(MeO)_2$-$C_6H_3$ | HBr | 208-9 |
| 25 | 2 | 3,4-$(OCH_2O)$-$C_6H_3$ | HBr | 196,5-197,5 |
| 26 | 2 | 4-MeO-$C_6H_4$ | HBr | 192-3 |
| 27 | 2 | 4-Me-$C_6H_4$ | HBr | 187-8 |

Les composés de l'invention (I) ont été soumis à des essais pharmacologiques qui ont montré leur activité antidépressive.

La toxicité des composés a été déterminée chez la souris par voie i.p. La DL 50 va de 100 à > 1000 mg/kg.

L'activité antidépressive a été déterminée slon le test de l'antagonisme vis à vis de la ptose réserpinique [Gouret C. et al., J. Pharmacol. (Paris) 8, 333-350 (1977)].

Les souris (mâles, CDI Charles River, France, 18-22 g) reçoivent simultanément les produits à étudier ou le solvant (voie i.p.), et la réserpine (4 mg/kg, voie s.c).

Soixante minutes plus tard, le degré de ptose palpébrale est estimé au moyen d'une échelle de cotation (0 à 4), pour chaque souris.

A chaque dose, la moyenne de cotation et le pourcentage de variation par rapport au lot contrôle, sont calculés.

Pour chaque produit, la DA 50, ou dose qui diminue de 50% le score moyen de ptose par rapport aux contrôles, est déterminée graphiquement.

La DA 50 varie de 1 à 5 mg/kg par voie i.p.

Les résultats pharmacologiques montrent que les composés de l'invention peuvent être utiles pour le traitement de la dépression.

Les composés de l'invention peuvent être présentés sous toute forme appropriée pour l'administration par voie orale ou parentérale, par exemple sous la forme de comprimés, dragées, gélules, solutions buvables ou injectables, etc... en association avec tout excipient approprié.

La posologie quotidienne peut aller de 5 à 200 mg.

**Revendications pour les Etats contractants:**
BE, CH, DE, FR, GB, IT, LI, LU, NL, SE

1. Dérivés phénéthylés de thiazole, et leurs isomères optiques répondant à la formule (I)

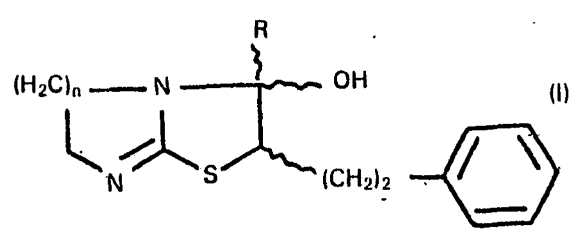

(I)

dans laquelle
n est 1 ou 2,
R est un radical naphthyle, phényle ou phényle portant un ou plusieurs substituants choisis dans le groupe constitué par les radicaux $C_{1-4}$alkyle, $C_{1-4}$ alkoxy, méthylènedioxy, $CF_3$ et les atomes d'halogène.
ainsi que leurs sels d'addition aux acides pharmaceutiquement acceptables.

2. Dérivés selon la revendication 1, dans lesquels R est un radical naphthyle, phényle ou phényle portant un ou deux substituants choisis parmi les radicaux méthyle, méthoxy, méthylènedioxy, $CF_3$ et les atomes de chlore, de brome et de fluor.

3. Dérivés selon la revendication 2, dans lesquels R est le radical phényle portant en position 3 ou en positions 3 et 4 un ou deux atomes de chlore ou groupes méthoxy.

4. Le phénéthyl-2 (chloro-3 phényl)-3 tétrahydro-2,3,6,7 5H-thiazolo[3,2-a]pyrimidinol-3 et ses sels pharmaceutiquement acceptables.

5. Le phénéthyl-2 (méthoxy-3 phényl)-3 tétrahydro-2,3,6,7 5H-thiazolo[3,2-a]pyrimidinol-3 et ses sels pharmaceutiquement acceptables.

6. Le phénéthyl-2 (chloro-3 phényl)-3 tétrahydro-2,3,5,6 imidazo[1,2-b]thiazolol-3 et ses sels pharmaceutiquement acceptables.

7. Le phénéthyl-2 (dichloro-3,4 phényl)-3 tétrahydro-2,3,5,6 imidazo[2,1-b]thiazolol-3 et ses sels pharmaceutiquement acceptables.

8. Procédé de préparation des composés selon la revendication 1, caractérisé en ce que l'on fait réagir l'imidazolidinethione-2 ou le tétrahydro-3,4,5,6 pyrimidine-thiol-2 de formule (II) ou (IIbis)

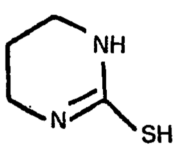
ou
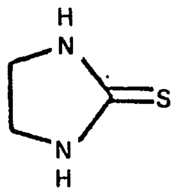

(IIbis) (II)

avec une cétone de formule (III)

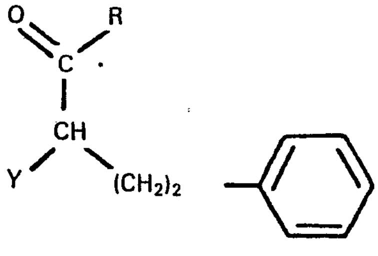
(III)

Y étant un atome de brome ou de chlore ou tout groupe labile.

9. Médicament caractérisé en ce qu'il contient un composé tel que spécifié dans l'une quelconque des revendications 1 à 7.

10. Composition pharmaceutique caractérisée en ce qu'elle contient à titre de principe actif un composé tel que spécifié dans l'une quelconque des revendications 1 à 7 en association avec tout excipient approprié.

**Revendication pour l'Etat contractant:** AT

Procédé de préparation de dérivés phénéthylés de thiazole et de leurs isomères optiques répondant à la formule (I)

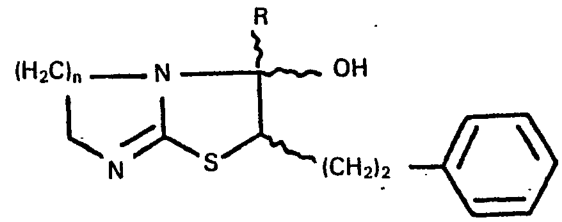

dans laquelle
n est 1 ou 2,
R est un radical naphthyle, phényle ou phényle portant un ou plusieurs substituants choisis dans le groupe constitué par les radicaux $C_{1-4}$alkyle, $C_{1-4}$alcoxy méthylènedioxy, $CF_3$ et les atomes d'halogène,
ainsi que de leurs sels d'addition aux acides pharmaceutiquement acceptables, caractérisé en ce que l'on fait réagir l'imidazolidinethione-2 ou le tétrahydro-3,4,5,6 pyrimidine-thiol-2 de formule (II) ou (IIbis)

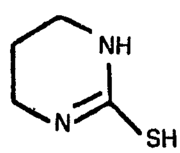
ou
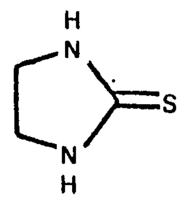

(IIbis) (II)

avec une cétone de formule (III)

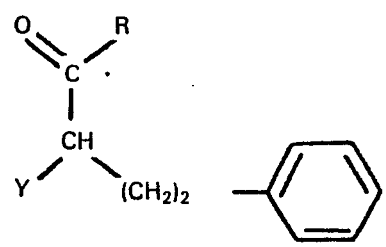
(III)

Y étant un atome de brome ou de chlore ou tout autre groupe labile.

**Patentansprüche für die Vertragsstaaten:**
BE, CH, DE, FR, GB, IT, LI, LU, NL, SE

1. Phenäthylderivate des Thiazols und ihre optischen Isomeren, entsprechend der Formel (I)

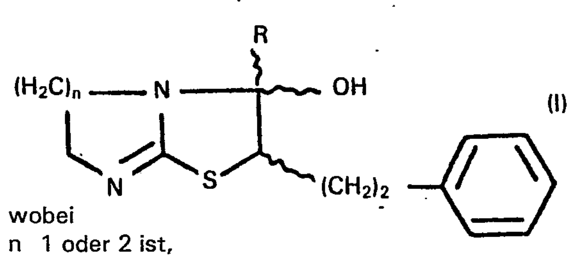
(I)

wobei
n 1 oder 2 ist,

R eine Naphthyl- oder Phenylgruppe oder eine Phenylgruppe mit einem oder mehreren Substituenten, ausgewählt aus der Gruppe: $C_{1-4}$-Alkylgruppen, $C_{1-4}$-Alkoxygruppen, Methylendioxy-, $CF_3$-Gruppen und Halogenatome bedeutet,
sowie ihre Additionssalze mit pharmazeutisch zulässigen Säuren.

2. Derivate nach Anspruch 1, wobei R eine Naphthyl- oder Phenylgruppe oder eine Phenylgruppe mit einem oder zwei Substituenten ausgewählt aus der Gruppe: Methyl-, Methoxy-, Methylendioxy-, $CF_3$-Gruppen und Chlor-, Brom- und Fluoratome, bedeutet.

3. Derivate nach Anspruch 2, wobei R eine Phenylgruppe ist, die in 3-Stellung oder in den Stellungen 3 und 4 ein oder zwei Chloratome oder Methoxygruppen trägt.

4. 2-Phenäthyl-3-(3-chlorphenyl)-2,3,6,7-tetrahydro-5H-thiazol[3,2-a]-pyrimidinol-3 und seine pharmazeutisch zulässigen Salze.

5. 2-Phenäthyl-3-(3-methoxyphenyl)-2,3,6,7-tetrahydro-5H-thiazol[3,2-a]-pyrimidinol-3 und seine pharmazeutisch zulässigen Salze.

6. 2-Phenäthyl-3-(3-chlorphenyl)-2,3,5,6-tetrahydro-imidazo[2,1-b]-thiazolol-3 und seine pharmazeutisch zulässigen Salze.

7. 2-Phenäthyl-3-(3,4-dichlorphenyl)-2,3,5,6-tetrahydro-imidazo[2,1-b]-thiazolol-3 und seine pharmazeutisch zulässigen Salze.

8. Verfahren zur Herstellung von Verbindungen nach Anspruch 1, dadurch gekennzeichnet, dass Imidazolidin-2-thion oder 3,4,5,6-Tetrahydro-pyrimidin-2-thiol der Formel (II) oder (IIb)

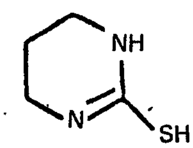
oder
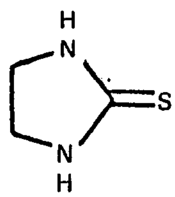

(IIbis) (II)

mit einem Keton der Formel (III)

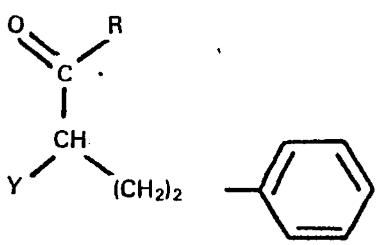
(III)

wobei Y ein Brom- oder Chloratom oder irgendeine labile Gruppe bedeutet, zur Reaktion gebracht wird.

9. Arzneimittel, dadurch gekennzeichnet, dass es eine Verbindung wie in einem der Ansprüche 1 bis 7 spezifiziert, enthält.

10. Pharmazeutische Zubereitung, dadurch gekennzeichnet, dass sie als Wirksubstanz eine Verbindung wie in einem der Ansprüche 1 bis 7 spezifiziert in Verbindung mit irgendeinem geeigneten Exzipiens enthält.

**Patentanspruch für den Vertragsstaat:** AT

Verfahren zur Herstellung von Phenäthylderivaten des Thiazols und ihrer optischen Isomeren, entsprechend Formel (I)

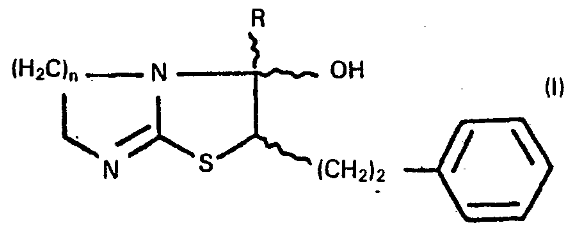
(I)

wobei
n 1 oder 2 ist,
R eine Naphthyl- oder Phenylgruppe oder eine Phenylgruppe mit einem oder mehreren Substituenten, ausgewählt aus der Gruppe: $C_{1-4}$-Alkyl-, $C_{1-4}$-Alkoxy-, Methylendioxy-, $CF_3$-Gruppen und Halogenatome, bedeutet,
sowie ihre Additionssalze mit pharmazeutisch zulässigen Säuren, dadurch gekennzeichnet, dass Imidazolidin-2-thion oder 3,4,5,6-Tetrahydro-pyrimidin-2-thiol der Formel (II) oder (IIb)

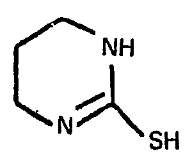
oder
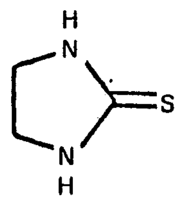

(IIbis) (II)

mit einem Keton der Formel (III)

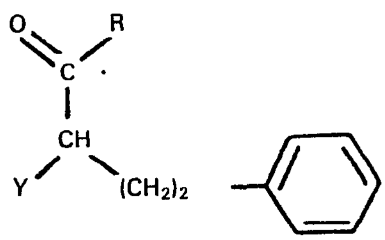
(III)

wobei Y ein Brom- oder Chloratom oder irgendeine andere labile Gruppe ist, zur Reaktion gebracht wird.

**Claims for the Contracting States:**
BE, CH, DE, FR, GB, IT, LI, LU, NL, SE

1. Phenethyl derivatives of thiazole, and optical isomers thereof, corresponding to formula (I)

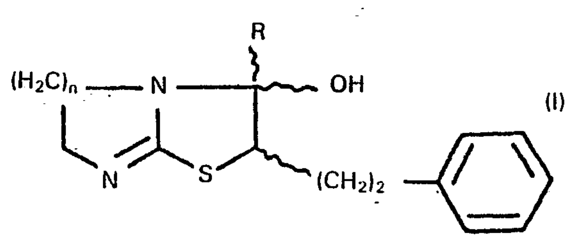
(I)

wherein

n is 1 or 2,
R is a naphthyl or phenyl radical, or a phenyl radical bearing one or more substituents selected from $C_{1-4}$-alkyl, $C_{1-4}$-alkoxy, methylenedioxy, $CF_3$ radicals and halogen atoms, and their pharmaceutically acceptable acid addition salts.

2. Derivatives according to claim 1, wherein R is a naphthyl radical, a phenyl radical or a phenyl radical bearing one or two substituents selected from the methyl, methoxy, methylenedioxy, $CF_3$ radicals and chlorine, bromine and fluorine atoms.

3. Derivatives according to claim 2, wherein R is the phenyl radical bearing on position 3 or positions 3 and 4 one or two chlorine atoms or methoxy groups.

4. 2-phenethyl-3-(3-chloro-phenyl)-2,3,6,7-tetrahydro-5H-thiazolo[3,2-a]pyrimidin-3-ol and its pharmaceutically acceptable salts.

5. 2-phenethyl-3-(3-methoxy-phenyl)-2,3,6,7-tetrahydro-5H-thiazolo[3,2-a]pyrimidin-3-ol and its pharmaceutically acceptable salts.

6. 2-phenethyl-3-(3-chloro-phenyl)-2,3,5,6-tetrahydro-imidazol[2,1-b]thiazol-3-ol and its pharmaceutically acceptable salts.

7. 2-phenethyl-3-(3,4-dichloro-phenyl)-tetrahydro-2,3,5,6-imidazo[2,1-b]thiazol-3-ol and its pharmaceutically acceptable salts.

8. Process for the preparation of the compounds according to claim 1, characterized in that imidazolidine-2-thione or 3,4,5,6-tetrahydro-pyrimidine-2-thiol of formula (II) or (IIbis)

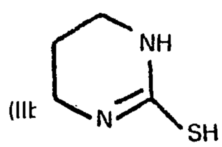 or 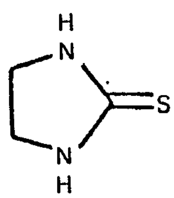

(IIbis) (II)

are reacted with a ketone of formula (III)

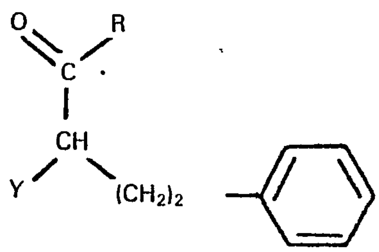

(III)

wherein Y is a bromine or chlorine atom or any labile group.

9. Medicament characterized in that it contains a compound as specified in any of claims 1 to 7.

10. Pharmaceutical composition characterized in that it contains as an active principle a compound as specified in anyone of claims 1 to 7 in association with any suitable excipient.

**Claim for the Contracting State:** AT

Process for the preparation of phenethyl derivatives of thiazole, and optical isomers thereof, corresponding to formula (I)

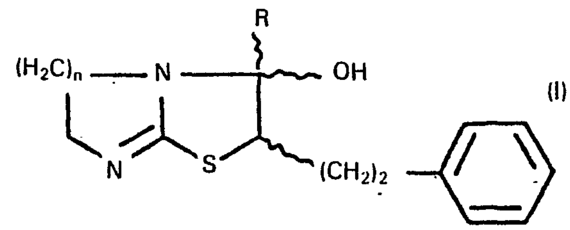

(I)

wherein
n is 1 or 2,
R is a naphthyl or phenyl radical, or a phenyl radical bearing one or more substituents selected from $C_{1-4}$-alkyl, $C_{1-4}$-alkoxy, methylenedioxy, $CF_3$ radicals and halogen atoms, and their pharmaceutically acceptable acid addition salts, characterized in that imidazolidine-2-thione or 3,4,5,6-tetrahydro-pyrimidine-2-thiol of formula (II) or (IIbis)

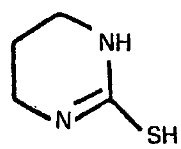 or 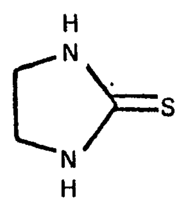

(IIbis) (II)

are reacted with a ketone of formula (III)

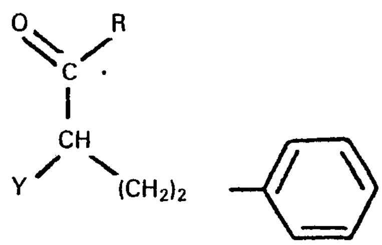

(III)

wherein Y is a bromine or chlorine atom or any labile group.